# EUROPEAN PATENT APPLICATION

(11) **EP 1 818 331 A1**
(43) Date of publication of application: **15.08.2007**
(21) Application number: 06101678.8
(22) Date of filing: 14.02.2006
(51) Int. Cl.: C07D 401/12

(54) **Process for the preparation of 2-[{4-(3-methoxypropoxy)-3-methylpyridin-2-yl}methylsulfinyl]-1H-benzimidazole substantially free of sulfone impurity**

(71) Applicant: EOS Eczacibasi Ozgun Kimyasal Urunler Sanayi Ve Ti Caret A.S., 80640 Istanbul (TR)
(72) Inventor: Adiyaman, Mustafa, 80640 Istanbul (TR); Akyuz, Yunus, 80640 Istanbul (TR)
(74) Representative: Sevinç, Erkan

(57) **Abstract**

A process for the preparation of rabeprazole substantially free of sulfone impurity is disclosed. The method comprises reacting 2-[{4-(3-methoxypropoxy)-3-methylpyridine-2-yl}methylthio]-1H-benzimidazole with an oxidizing agent and removing the unreacted sulfide compounds and sulfone impurities from the medium by means of an extraction and two additional crystallization steps. The unreacted compounds are removed with the organic layer of said extraction step whereas the impurities are removed with the additional crystallization steps wherein rabeprazole free base and impurities are treated in the same phase. Preparation of rabeprazole sodium salt is also covered within the scope of the present invention. Product of the previous crystallization steps is dissolved in methanolic sodium hydroxide and obtained solution is concentrated. Sodium salt of rabeprazole is precipitated by way of adding the residue into diethyl ether. Both rabeprazole and its sodium salt are obtained with sulfone impurity percentage around 0.08 %.

## Description

### Technical Field of the Invention

The present invention relates to a method for synthesis of 2-[{4-(3-methoxypropoxy)-3-methylpyridine-2-yl}methylsulfinyl]-1H-benzimidazole in a form which is substantially free of sulfone impurities. Obtaining pharmaceutically acceptable salts, particularly sodium salts thereof are also disclosed.

### Background of the Invention

2-[{4-(3-methoxypropoxy)-3-methylpyridine-2-yl}methylsulfinyl]-1H-benzimidazole, hereinafter referred with its common commercial name Rabeprazole is used as a proton pump inhibitor (PPI) that block the production of acids in the stomach. In general practice rabeprazole, like other proton pump inhibitors, is used for the treatment of conditions such as ulcers, gastroesophageal reflux disease (GERD) and Zollinger-Ellison Syndrom, mainly associated with acid formation in the stomach.

Benzimidazole type pyridine derivatives and their production methods are widely disclosed in the art and have been under active development in the past years. Rabeprazole and such improved methods of its preparation are disclosed for instance in WO 03/08406, EP 1 300 406 and US 5,045,552. Rabeprazole, having the molecular formula; 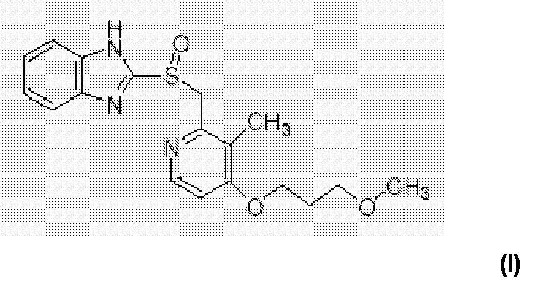 is typically synthesized by oxidizing 2-[{4-(3-methoxypropoxy)-3-methylpyridine-2-yl}methylthio]-1H-benzimidazole with a suitable oxidizing agent in the presence of a solvent. During this procedure, further oxidation of the sulfoxide of the rabeprazole causes the problem, so called "over-oxidation" resulting sulfone impurities in the final product which is hard to control with classical methods in the state of the art.

WO 03/08406, as one of the most relevant documents, discloses a process in which the main object appears in the elimination of such sulfone impurities. Removal of unreacted reactants and sulfone impurities is carried out by a double step extraction wherein sulfone impurities are removed with the water layer of the first extraction and unreacted sulfides are removed with the organic layer of the second extraction. The process proposed in this document provides relatively lower amount of sulfone compounds in the out coming final product compared to the conventional methods found in the art. At this point, however, removal of formed sulfones in the first extraction with the water layer still carries disadvantages in that sulfone impurities may still form up within the reaction medium even after removal of the same in the first extraction. Furthermore, using more than one extraction step makes the process relatively more complex and costly to operate. It is reported in WO 03/08406 that product obtained by such process contains around 0.8 % or lower amount of sulfone impurities.

The present invention solves entire of such problems through use of single extraction step, in which unreacted sulfide compounds are firstly removed with the organic phase, and two additional crystallization steps wherein the product and sulfone impurities are separated in the same phase. This primarily provides higher yield of product with lower percentage of sulfone impurities, typically lower than 0.08 % i.e. ten times less than that of conventional techniques.

US 5,045,552 discloses pyridine derivatives having anti-ulcerative activity and a process relating thereto. Example 32 of the specification particularly describes a method of producing rabeprazole free base and example 33 describes a method for preparing its sodium salt form. In the description part, sulfone compounds are handled not as an impurity but as an object of the disclosure. Examples 32-33 expressed above, and remainder of the specification of US 5,045,552 neither discloses a process for eliminating impurities i.e. sulfone compounds, nor a process for removal of formed impurities. After an oxidation reaction which occurs at -45°C, extraction by use of dichloromethane takes place in the process for removing unreacted sulfide compounds and obtaining the product in the aqueous phase. Said aqueous layer is washed with dichloromethane at pH 11 and dried over magnesium sulfate and distilled to remove dichloromethane. When the process is inspected carefully many drawbacks, making it inefficient and costly to operate, appear in many aspects. Temperature of the oxidation reaction which is reported as typically -45°C, is a considerably low temperature and requires large amounts of energy or cooling material to maintain in the reaction medium. Furthermore, product is obtained directly right after an extraction step which makes the ratio of sulfone impurities quite high in the final product. According to the laboratory scale trials of this method, the inventors of the present application noted that sulfone impurities as high as ranging from 0.58 % to 5.54 %, are unavoidably obtained with this conventional method. In addition, dichloromethane is removed from the product by way of distillation which may readily damage the molecular structure of rabeprazole free base or its salts. Still a further drawback of this method, lies in that the pH value of the aqueous layer which is adjusted to 11, enhances the formation of sulfone impurities. It should be noted that oxidation reaction of the present invention is carried out at a much higher temperature, typically around -10°C. No additional distillation step is required during the entire process. Those skilled in the art will readily appreciate that elimination of such drawbacks makes the overall process cost effective and efficient along with the higher quality of the final product which is almost free of sulfone impurities.

EP 1 300 406 discloses processes for producing pyridine compounds in general and particular methods for producing rabeprazole free base are exemplified in examples 1-8, 11-20 and 22-26. In accordance with its disclosure, sulfone impurities are eliminated by changing the oxidation reaction mechanism wherein halogenated hydrocarbon solvent is not employed for the purpose of oxidation. Instead, N-halosuccinimide, a 1,3-dihalo-5,5-dimethylhydantoin or a dichloroisocyanurate is used in the presence of an inorganic base. It has been noted by the inventors of the present application that aforementioned oxidation agents may eliminate over oxidation of sulfide compounds, whereas it is known that these compounds are unable to present activity as much as conventional halogenated oxidizing agents, with the obvious result that the overall process yield lowers considerably. Furthermore, safety and health related problems may arise particularly for the case of dichloroisocyanurates.

The present invention proposes that sulfone compounds are separated from the product when they are in the same phase after removal of the unreacted compounds with the first extraction. Also process parameters can be adjusted to eliminate the drawbacks of the previously known methods mentioned briefly above. The final product having a sulfone impurity level lower than 0.08 %, which is nearly one tenth of the amount of conventional methods can be obtained. Further details of above mentioned solutions are given widely below in the detailed description.

### Summary of the Invention

The present invention relates to an improved method of producing rabeprazole free base and sodium salts thereof, which are substantially free of sulfone impurities. Reaction mechanisms employed in the process for producing rabeprazole in general comprises the reaction of 2-[{4-(3-methoxypropoxy)-3-methylpyridine-2-yl}methylthio] -1H-benzimidazole with an oxidizing agent. This oxidation reaction is conducted at a temperature ranging preferably from -12 to -8°C in the presence of a solvent or mixture of solvents. An extraction step after completion of the oxidation reaction is carried out by use of a solvent such as dichloromethane after which the unreacted 2-[{4-(3-methoxypropoxy)-3-methylpyridine-2-yl}methylthio]-1H-benzimidazole compounds are removed with the organic phase. The aqueous solution obtained after removing said organic phase contains primarily the rabeprazole free base along with its sulfone impurities.

The product and said impurities are treated in the same phase during the rest of the process in which additional crystallization steps for obtaining rabeprazole free base in substantially pure form are employed. One of the advantages of the present invention appears with the elimination of a requirement of separating the product from the sulfone impurities through different phases as per mentioned in the background of the invention. Crystallization steps of the process are applied sequentially after the extraction step for which inventors of the present invention found that pH around 10.2 is the critical range for obtaining the product with minimum amount of sulfone compounds.

Also obtaining pharmaceutically acceptable salts, preferably sodium salt of rabeprazole, is another object of the present invention. Outcoming crystallized product of the previous crystallization step is dissolved in a suitable solvent and the solution so obtained is concentrated. The residue is treated with diethyl ether in order to precipitate sodium salt of rabeprazole which is recovered in substantially pure form. Further details and objects of the invention are explained below in the rest of the description and claims.

### Objects of the Invention

The present invention relates mainly to a method of obtaining rabeprazole and its sodium salt in substantially pure form.

A first object of the present invention is to provide a method for producing rabeprazole free base and its sodium salt where amount of sulfone impurities in the bulk product is substantially around 0.08 %.

Another object of the present invention is to provide a process in which the need for employing an additional extraction step for removal of sulfone impurities is eliminated.

A further object of the present invention is to provide a process wherein rabeprazole free base and sulfone impurities thereof are separated by way of additional crystallization steps when they are in the same phase.

A further object of the invention is to provide a process having relatively higher yield and product purity compared to existing methods found in the art.

Still a further object of the present invention is to provide a process in which the oxidation reaction is carried out at relatively high temperatures providing cost effective operation.

Yet another object of the present invention is to provide a process which is safe with respect to health and environment.

### Detailed Description of the Invention

Rabeprazole and other benzimidazole type pyridine derivatives are conventionally produced by way of an oxidation reaction and the product is recovered thereafter. Reacting 2-[{4-(3-methoxypropoxy)-3-methylpyridine-2-yl}methylthio]-1H-benzimidazole having the structural formula; 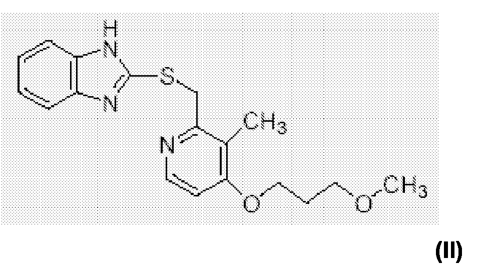 with an oxidizing agent, particularly with a halogenated oxidizing compound such as m-Chloroperbenzoic acid having the chemical formula; 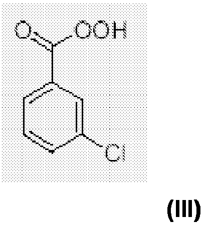 for obtaining the rabeprazole free base is the well known oxidation reaction mechanism used in the art. During this reaction, sulfide compound given with the formula (II) unavoidably further oxidizes and forms sulfone impurities as a side product. Certain references in the sate of the art either ignore this problem or bring solutions such as a further separation step taking said sulfones in one phase and taking the product in another phase and finally removing the sulfone phase from the mixture medium. Such a method may be utilized for removal of those sulfones however, the final product of the process still contains impurities of around 1.0 %, which is more than ten times of that proposed by the present invention.

Oxidation reaction of the present invention is carried out in accordance to the reaction mechanism given above, i.e. by way of dissolving sulfide compound of formula (II) in a suitable solvent selected from the group of dichloromethane, diethyl ether and chloroform, preferably in the mixture of dichloromethane and diethyl ether and reacting the same with an oxidizing agent preferably with m-chloroperbenzoic acid of formula (III). The oxidizing agent can also well be selected from the group consisting of hydrogen peroxide, permanganates and peroxy monosulfate. This reaction is carried out at a temperature ranging from -20°C to -5°C, more preferably at -12 to - 8°C with or without a catalyst. The present invention hereby eliminates the need for very low reaction temperatures which is important for the costs of the operation.

After completion of the reaction, triethylamine is added to the reaction mixture and medium is heated to room temperature. An aqueous solution of an organic or inorganic base is added to the medium and this mixture is extracted with a suitable solvent selected from the group of dichloromethane, diethyl ether and chloroform, preferably with dichloromethane to remove unreacted sulfides of formula (II). Impurities and the rabeprazole free base remains in the aqueous layer whereas said sulfides are removed with the organic phase. Removing first the unreacted substances with this extraction step brings some advantages such as elimination of uncontrolled further oxidation and separation of the product from impurities more efficiently in the later stages.

Resulting aqueous phase is treated preferably with aqueous ammonium acetate for adjusting the pH to a range 9.5 to 11.0, more preferably to a range 10.0 to 10.5 and most preferably to 10.2 ± 0.05. This is the critical pH value of the mixture which is thought to be the most ideal range for obtaining minimum impurity in the product. Solution is stirred for a duration of 5 to 20 hours, most preferably 8 to 14 hours for crystallizing the product and removing sulfone impurities. Consequently, an off-white crystal is obtained and separated by means of filtration. Thereby, first crystallization step for removal of the impurities is completed.

Wet cake of the previous step is dissolved in aqueous solution of a base and resulting solution is stirred for a short while, generally less than 60 minutes. pH of this solution is typically around 13.0 which is out of the optimum range of operation. Therefore, pH is adjusted to the most preferable value ranging 9.5 to 11.0, more preferably to a range 10.0 to 10.5 and most preferably to 10.2 ± 0.05 as in the previous crystallization step. Likewise, the solution obtained thereafter is stirred for a duration of 5 to 20 hours, more preferably 8 to 14 hours for crystallizing the product and removing the sulfone impurities. A yellowish white crystal structure is obtained and same is separated by filtration. As a consequence of this second crystallization step, product is separated from the sulfone impurities, wherein said impurities constitute less than 0.08 % of the product. Said base mentioned above can be an inorganic base such as sodium hydroxide, potassium hydroxide and sodium carbonate, or an organic base such as ammonia.

Wet cake of the previous step is dissolved in a suitable solvent, preferably in dichloromethane or chloroform and so formed solution is crystallized from diethyl ether to obtain rabeprazole free base having the structural formula (I); 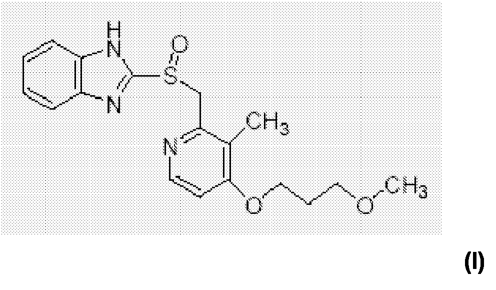 Stirring operations employed in the above mentioned crystallization steps have also a particular importance due to the reason that impurities and the product are in the same phase, therefore efficient and adequate duration of stirring should be provided for obtaining the desired product in substantially pure form.

It is one of the objects and advantages of the present invention that sodium salt of rabeprazole may readily be obtained by means of the additional process steps applied right after obtaining the rabeprazole free base. For that purpose, rabeprazole free base obtained in substantially pure form in the previous step is dissolved in aqueous solution of sodium hydroxide, or more preferably in methanolic sodium hydroxide. Resulting solution is concentrated and added to diethyl ether for precipitating 2-[{4-(3-methoxypropoxy)-3-methylpyridine-2-yl}methylsulfinyl]-1H-benzimidazole sodium salt of formula (IV).

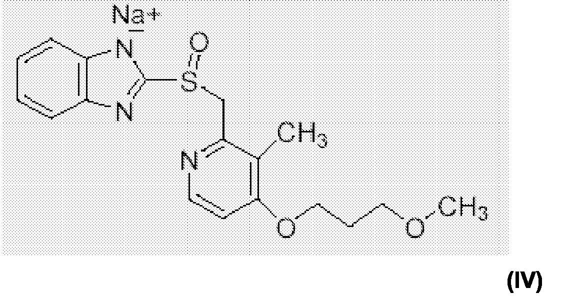

### Example 1

### Preparation of 2-[{4-(3-methoxypropoxy)-3-methylpyridine-2-yl}methylsulfinyl]-1H-benzimidazole (Rabeprazole Free Base)

30.0 g (8.74 mmol) of 2-[{4-(3-methoxypropoxy)-3-methylpyridine-2-yl}methylthio]-1 H-benzimidazole is dissolved in 600.0 ml of dichloromethane and 50.0 ml of diethyl ether mixture and 23.3 g of 70.0 % m-choloroperbenzoic acid is added to this solution at -10°C in 5.0 minutes. So formed solution is stirred for an hour until the oxidation reaction is completed. 12.0 g of triethylamine is added to the reaction mixture which is heated to room temperature thereafter. 480.0 ml of 1 N aqueous solution of NaOH is added to the reaction mixture and the same is stirred for 45 minutes. The obtained mixture is extracted with 150.0 ml of dichloromethane thrice to remove the unreacted 2-[{4-(3-methoxypropoxy)-3-methylpyridine-2-yl}methylthio]-1 H-benzimidazole. Organic phases are removed for this purpose. pH value of the aqueous phase is measured as 13.44. For bringing this value to an optimum pH value of 10.2 ± 0.05, 119.0 ml of an aqueous solution of 2M ammonium acetate is admixed to the medium. The solution is stirred over night at room temperature after which crystallization occurs. The white crystals are separated by way of filtration for the further crystallization step. Obtained wet cake is dissolved in 100.0 ml of 1 N NaOH and stirred for 0.5 h wherein pH is measured as 13.35. pH is adjusted to 10.2 ± 0.05 with addition of 2M ammonium acetate into the medium. The solution is stirred over night at room temperature and crystallization is completed. So formed yellowish-white crystal product is separated by filtration. 13.5 g of wet cake was dissolved in 100.0 ml of dichloromethane and the solution obtained thereby is crystallized from diethyl ether to obtain 11.0 g of 2-[{4-(3-methoxypropoxy)-3-methylpyridine-2-yl}methylsulfinyl]-1H-benzimidazole. Percentage of product purity is 99.61 % and sulfone impurity in the bulk product is 0.08 %.

### Example 2

### Preparation of 2-[{4-(3-methoxypropoxy)-3-methylpyridine-2-yl}methylsulfinyl]-1H-benzimidazole Sodium Salt (Rabeprazole Sodium Salt)

The product of example 1 is dissolved in 53.0 ml of 0.5 N and 0.1 % aqueous solution of methanolic sodium hydroxide. Obtained solution is concentrated and the residue is added to diethyl ether dropwise to precipitate 2-[{4-(3-methoxypropoxy)-3-methylpyridine-2-yl}methylsulfinyl]-1H-benzimidazole sodium salt. Percentage of product purity is 99.83 % and sulfone impurity in the bulk product is 0.07 %.

The process of the present invention does not require additional complicated separation steps such as extraction, distillation or considerably low operation temperatures as mentioned above in the background of the invention. The product which is almost free of sulfone impurities is successfully obtained in a cost effective manner. Further advantages of the process are apparent to those skilled in the art and the invention is not limited to specific components or examples mentioned herewith.

## Claims

1. A process for producing 2-[{4-(3-methoxypropoxy)-3-methylpyridine-2-yl} methylsulfinyl]-1H-benzimidazole free base or its sodium salt comprising the steps of:
a) dissolving 2-[{4-(3-methoxypropoxy)-3-methylpyridine-2-yl}methylthio]-1H-benzimidazole in the presence of a suitable solvent,
b) reacting the same with an oxidizing agent,
**characterized in that** the process further comprises the steps of:
c) adding an aqueous solution of a base to the reaction mixture,
d) extracting said reaction mixture with a suitable solvent for removing the unreacted 2-[{4-(3-methoxypropoxy)-3-methylpyridine-2-yl}methylthio]-1H-benzimidazole compounds with the organic phase,
e) crystallizing the aqueous layer of the previous extraction step by means of stirring the same at pH 9.5 to 11.0, more preferably at pH 10.0 to 10.5,
f) dissolving the wet cake of the previous crystallization step in an aqueous solution of a base and re-crystallizing the resulting solution by means of stirring at pH 9.5 to 11.0, preferably at pH 10.0 to 10.5.

2. A process for producing the sodium salt of 2-[{4-(3-methoxypropoxy)-3-methylpyridine-2-yl}methylsulfinyl]-1H-benzimidazole as set forth in Claim 1, wherein the process further comprises the steps of ;
g) dissolving the free base product of claim 1 in a suitable solvent,
h) concentrating the obtained solution and adding the resulting residue into diethyl ether for precipitating the sodium salt form of the product.

3. A process according to claim 1 wherein solvents of step (a) and (d) are selected from the group consisting of dichloromethane, diethyl ether and chloroform.

4. A process according to claim 1 wherein said oxidizing agent is selected from the group consisting of m-chloroperbenzoic acid, hydrogen peroxide, peroxy monosulfate and permanganates.

5. A process according to claim 1 wherein said base of step (c) and (f) is preferably an inorganic base selected from the group consisting of sodium hydroxide, potassium hydroxide and sodium carbonate, or an organic base such as ammonia.

6. A process according to claim 1 wherein the crystallization steps of (e) and (f) are carried out at pH ranging from 10.0 to 10.5, more preferably at pH 10.2 ± 0.05.

7. A process according to claim 1 wherein stirring of the crystallization mediums of step (e) end (f) are carried out for a duration of from 5 to 20 hours, more preferably from 8 to 14 hours.

8. A process according to claim 1 wherein the oxidation reaction is carried out at a temperature ranging from -20 to -5°C, more preferably from -12 to -8°C.

9. A process according to claim 1 wherein triethylamine is added to the reaction medium upon completion of the oxidation reaction.

10. A process according to claim 2 wherein the solvent is a solution of sodium hydroxide, and preferably methanolic sodium hydroxide.

## Amended claims

### Amended claims in accordance with Rule 86(2) EPC.

**1.** A process for producing 2-[{4-(3-methoxypropoxy)-3-methylpyridine-2-yl} methylsulfinyl]-1 H-benzimidazole free base or its sodium salt comprising the steps of:
a) dissolving 2-[{4-(3-methoxypropoxy)-3-methylpyridine-2-yl}methylthio]-1 H-benzimidazole in the presence of a suitable solvent,
b) reacting the same with an oxidizing agent,
c) adding an aqueous solution of a base to the reaction mixture,
d) extracting said reaction mixture with a suitable solvent for removing the unreacted 2-[{4-(3-methoxypropoxy)-3-methylpyridine-2-yl}methylthio]-1 H-benzimidazole compounds with the organic phase, **characterized in that** the process further comprises the steps of:
e) crystallizing the aqueous layer of the previous extraction step by means of stirring the same at pH 9.5 to 11.0,
f) dissolving the wet cake of the previous crystallization step in an aqueous solution of a base and re-crystallizing the resulting solution by means of stirring at pH 9.5 to 11.0.

**2.** A process for producing the sodium salt of 2-[{4-(3-methoxypropoxy)-3-methylpyridine-2-yl} methylsulfinyl]-1 H-benzimidazole as set forth in Claim 1, wherein the process further comprises the steps of ;
g) dissolving the free base product of claim 1 in a suitable solvent,
h) concentrating the obtained solution and adding the resulting residue into diethyl ether for precipitating the sodium salt form of the product.

**3.** A process according to claim 1 wherein solvents of step (a) and (d) are selected from the group consisting of dichloromethane, diethyl ether and chloroform.

**4.** A process according to claim 1 wherein said oxidizing agent is selected from the group consisting of m-chloroperbenzoic acid, hydrogen peroxide, peroxy monosulfate and permanganates.

**5.** A process according to claim 1 wherein said base of step (c) and (f) is preferably an inorganic base selected from the group consisting of sodium hydroxide, potassium hydroxide and sodium carbonate, or an organic base such as ammonia.

**6.** A process according to claim 1 wherein the crystallization steps of (e) and (f) are carried out at pH ranging from 10.0 to 10.5.

**7.** A process according to claim 1 wherein the crystallization steps of (e) and (f) are carried out at pH 10.2 ± 0.05.

**8.** A process according to claim 1 wherein stirring of the crystallization mediums of step (e) end (f) are carried out for a duration of from 5 to 20 hours, more preferably from 8 to 14 hours.

**9.** A process according to claim 1 wherein the oxidation reaction is carried out at a temperature ranging from -20 to -5°C, more preferably from -12 to -8°C.

**10.** A process according to claim 1 wherein triethylamine is added to the reaction medium upon completion of the oxidation reaction.

**11.** A process according to claim 2 wherein the solvent is a solution of sodium hydroxide, and preferably methanolic sodium hydroxide.
